Europäisches Patentamt

European Patent Office

Office européen des brevets

(19)

(11) Veröffentlichungsnummer: **0 190 610 B1**

# (12) EUROPÄISCHE PATENTSCHRIFT

(45) Veröffentlichungstag der Patentschrift: **24.04.91**

(21) Anmeldenummer: **86100835.7**

(22) Anmeldetag: **22.01.86**

(51) Int. Cl.⁵: **C12P 7/06, C12F 3/10**

(54) **Verfahren zur Gewinnung von Alkohol und proteinangereicherter Schlempe aus zucker-, stärke- und/oder zellulosehaltigen Rohstoffen.**

(30) Priorität: **24.01.85 DE 3502217**

(43) Veröffentlichungstag der Anmeldung:
**13.08.86 Patentblatt 86/33**

(45) Bekanntmachung des Hinweises auf die
Patenterteilung:
**24.04.91 Patentblatt 91/17**

(84) Benannte Vertragsstaaten:
**AT BE DE FR GB IT LU NL SE**

(56) Entgegenhaltungen:
**DE-C- 358 118**
**GB-A- 2 089 836**

**CHEMICAL ABSTRACTS, Band 97, Nr. 23, Dezember 1982, Seite 456, Nr. 196857e, Columbus, Ohio, US; & BR - A - 80 08 389 (G. ANDERLE) 03.08.1982**

(73) Patentinhaber: **Wilke, Engelbart**
**Welsburg**
**W-2879 Brettorf(DE)**

(72) Erfinder: **Wilke, Engelbart**
**Welsburg**
**W-2879 Brettorf(DE)**

(74) Vertreter: **Ninnemann, Detlef, Dipl.-Ing.**
**Patentanwälte Maikowski & Ninnemann Xantener Strasse 10**
**W-1000 Berlin 15(DE)**

**Beschreibung**

Die Steigerung der Wirtschaftlichkeit bei der Gewinnung von Alkohol aus landwirtschaftlichen Stoffen ist im Hinblick auf die Nutzung des Alkohols als Energiequelle oder Chemierohstoff von großer Wichtigkeit. Die kostengünstige Schlempenutzung und Schlempeverwertung stellt dabei ein Hauptproblem dar.

Die DE-OS 30 23 874 beschreibt ein Verfahren, bei dem die nach Entfernen der Grobverunreinigungen mit reduzierter Trockensubstanz anfallende Schlempe ohne weiter Reinigung mehrfach in den Gärprozeß zurückgeführt wird, bis der Trockensubstanzgehalt der Schlempe auf bis zu 30 % Trockensubstand angestiegen ist. Auf diese Weise soll eine konzentrierte Schlempe gewonnen werden. Eine weitere Schlemperückführung ist wegen osmotischer und andrer Einflüsse auf die Gärorganismen nicht möglich. Es hat sich aber gezeigt, daß bei auch nur einmaliger Rückführung einer solchen Schlempe eine Minderung der Alkoholausbeute in der Größenordnung von 1 % der Gesamtausbeute eintritt. Wegen der hohen Rohstoffkosten wird daher dieses Verfahren unwirtschaftlich.

Nutzungsmöglichkeiten der Schlempe, wie sie beispielsweise aus der DE-OS 24 42 533 bekannt sind, bei denen bestimmte Mikroorganismen die gelösten Schlempebestandteile zu ungelösten umwandeln, die dann als Futtermittel genutzt werden sollen, stoßen wegen der ungelösten Frage der Infektion mit pathologischen Keimen, die in das Futter gelangen können, auf großen Widerstand.

Es ist schon versucht worden, Mikroorganismen, beispielsweise Bakterien, mit teilgereinigter Schlempe als Substrat unter Zusatz von Nährsalzen und Luft zu züchten und die sterilisierten und aufgeschlossenen Mikroorganismen bei der Backhefezucht wieder zu verwenden. Da aber nicht verhindert werden kann, daß mit den sterilisierten Mikroorganismen auch gefährliche Fremdkeime in die Hefe gelangen, scheiterte dieses Vorhaben.

Aus der DE-C-358 118 ist ein Verfahren zur Gewinnung von Alkohol und proteinangereicherter Schlempe aus zellulosehaltigen Rohstoffen bekannt, bei dem die bei der Alkoholabtrennung anfallende Schlempe biologisch mit Pilzen behandelt wird, dabei in der Schlempe gelöste organische Stoffe abgebaut und die Pilze vermehrt werden sowie die so behandelte Schlempe als Nährstoffbestandteil wiederum für die Hefegärung benutzt wird.

Es hat sich jedoch herausgestellt, daß Monokulturen wie Pilze die in der Schlempe gelösten organischen Stoffe nicht weit genug abbauen können, so daß die Schlempe nur in geringem Umfange als Nährstoffbestandteil für die Hefegärung benutzt werden kann, da die organischen Reststoffe einen hohen Hemmeffekt auf die Entwicklung der Hefe haben.

Aufgabe der vorliegenden Erfindung ist es, ein Verfahren zur Gewinnung von Alkohol und proteinangereicherter Schlempe aus zucker-, stärke- und/oder zellolosehaltigen Rohstoffen der eingangs genannten Gattung zu schaffen, bei dem die Schlempe vollständig als Nährstoffbestandteil für die Hefegärung wiederverwendet und auch bei einer Zufuhr weiterer organischer Stoffe eine optimale Hefegärung gewährleistet wird.

Diese Aufgabe wird durch die kennzeichnenden Merkmale des Anspruchs 1 gelöst.

Die erfindungsgemäße Lösung stellt zum einen sicher, daß durch die Verwendung von Belebtschlamm, d.h. Mischkulturen von Bakterien die organischen Stoffe optimal abgebaut werden können, so daß ein minimaler Sauerstoffbedarf nach der biologischen Behandlung auftritt und damit die Hefegärung optimal verlaufen kann.

Vorteilhafte Ausgestaltungen und Weiterbildungen der Erfindung sind in den Unteransprüchen gekennzeichnet.

Die durch das erfindungsgemäße Verfahren erzielten Ergebnisse waren überraschend, da allgemein bekannt war, daß bei der Alkoholgärung schon geringe Infektionen mit Bakterien drastische Störungen und Ausbeuteverluste bewirken. Unerwarteterweise zeigte sich bei einem Versuch, bei dem Getreidemehl anstatt mit Wasser, mit Belebtschlamm aus einer biologischen Behandlung von Tiergülle angeteigt und zu süßer Maische verarbeitet wurde, daß bei der Alkoholgärung unter mikroaeroben Bedingungen nicht nur Infektionen mit Fremdkeimen keine Rolle spielten, sondern daß auch die Ausbeute an Alkohol erhöht war. Noch positiver waren die Ergebnisse, wenn der Belebtschlamm auf teilgereinigter Schlempe unter Zusatz von Nährsalzen oder Tiergülle bzw. Flüssiggülle und starker Belüftung erzeugt wurde. Dabei kann die Proteinausbeute der Schlempe wesentlich gesteigert werden. Wenn das biologische Behandlungsverfahren für die von Grobstoffen befreite Schlempe so gesteuert wird, daß nur noch ein kleiner Rest von gelösten organischen Stoffen, gemessen beispielsweise als $BSB_5$ oder CSB, in der behandelten Schlempe verbleibt, kann die entstehende flüssige Masse beliebig oft zur Herstellung süßer Maischen verwendet werden, ohne daß es zu irgendwelchen Störungen kommt.

Es wurde gefunden, daß beim erfindungsgemäßen Verfahren in einer Brennerei, die Alkohol beispielsweise aus Getreide erzeugt, nur so viel Wasser als Reinwasser oder in Form von Tiergülle bzw. Flüssiggülle bei der Bereitung der Maische für die Alkoholgärung eingesetzt werden muß, wie durch Verdunsten und Entfernen mit den Grobstoffen der Schlempe verloren geht.

Durch den Zusatz von Nährsalzen oder Tiergülle bzw. Flüssiggülle bei der biologischen Behandlung der Schlempe kommt es zu einer hohen Produktion von Mikroorganismenprotein. Das dabei anfallende Protein wird bei der Alkoholgärung zum Teil für die Alkoholbildung, zum Teil für die Gärorganismenbildung ausgenutzt und zum Teil durch den entstehenden Alkohol sowie durch die Verhältnisse in der gärenden Maische und der Alkoholabtrennung ausgefällt. Daraus ergibt sich, daß dieses Protein bei der Entfernung der Schlempe-Grobstoffe beispielsweise in einem Dekanter mit anfällt und zu einer Proteinanreicherung der Dickschlempe führt. Die gelösten organischen Bestandteile der Schlempe können durch Verwendung eines Belebtschlammes, d.h. eines Mikroorganismengemisches, in einfacher Weise weitgehend in Mikroorganismenmasse umgewandelt werden.

Es wurde nun gefunden, daß diese Mikroorganismenmasse, wenn sie bei der Herstellung der Maische verwendet wird, d.h. wenn sie einem mechanischen, thermischen, enzymatischen Prozeß zusammen mit den zuckerhaltigen und/oder stärkehaltigen und/oder zellulosehaltigen Rohstoffen unterworfen wird, weder eine Infektionsgefahr für die gärende Maische, noch eine Gefahr für die Alkoholausbeute darstellt. Im Gegenteil, Infektionen spielten keine Rolle, wenn bei der Alkoholgärung durch ständige geringe Belüftung der gärenden Maische mikroaerobe Bedingungen vorlagen. Die Alkoholausbeute war sogar erhöht. Dabei kann, falls gewünscht, die behandelte Dünnschlempe unbegrenzt wiederverwendet werden.

Wenn die Alkoholgärung absatzweise unter Zuhilfenahme von kleinen Mengen gärender, noch belüfteter vergorener Maische durchgeführt wird, werden mit steigender Zahl der Wiederholungen, nicht wie sonst üblich schlechtere Ergebnisse, sondern zunehmend bessere Alkoholausbeuten erzielt.

Besonders hohe Ausbeuten an Alkohol aus stärkehaltigen oder zellulosehaltigen Rohstoffen werden dann erzielt, wenn die Verzuckerungsenzyme - anders als bisher in einen Maischbottich gegeben werden, um dort vor der Gärung schon einen Teil Zucker zu bilden - erst dann in den Gärbottich dosiert werden, wenn die Glucose- bzw. Maltosekonzentration oder Cellobiosekonzentration unter 5 Millimol pro Liter gärende Maische gefallen ist. Dabei ist zu beachten, daß bei der Befüllung des Gärbottichs schon vorzugsweise 2% des Endvolumens als Impfmaische ausreichen und daß erst etwa 10 Stunden nach dem Beginn der Befüllung mit verflüssigter, aber unverzuckerter Maische mit der Dosierung von Verzuckerungsenzymen in den Gärbottich begonnen werden muß.

Wo mit den Rohstoffen schon eine größere Wassermenge anfällt und deshalb kein vollständiges Flüssigkeitsrecycling möglich ist, wie beispielsweise bei Kartoffeln, kann beim erfindungsgemäßen Verfahren nur ein Teil der biologisch behandelten Flüssigkeit zusammen mit der darin angereicherten Mikroorganismenmasse bei der Herstellung der süßen Maische verwendet werden. In diesen Fällen entsteht Abwasser mit einem gegenüber bekannten Verfahren verminderten Gehalt an organischen Belastungen, das in bekannter Weise weiterbehandelt werden kann.

Wird ein Teil der benötigten Nährstoffe, beispielsweise Harnstoff, nicht in den Belebtschlammfermenter, sondern bei der Alkoholgärung zugesetzt, steigt damit die Proteingesamtausbeute in der Dickschlempe an und der Anteil, der im Belebtschlammfermenter produziert werden muß, sinkt erheblich. Überraschenderweise erfolgt diese Ausbeutesteigerung beim Schlempeprotein nicht auf Kosten der Alkoholausbeute.

Bei der biologischen Behandlung der Dünnschlempe durch die Mikroorganismen werden Nährsalze, Spurenelemente, Sauerstoff und ggf. andere Stoffe in solcher Weise und Menge zugesetzt, daß ein Maximum an Mikroorganismen entsteht. Der pH-Wert wird vorzugsweise auf 6 bis 8 eingestellt. Ein Teil der benötigten Nährstoffe für die Belebtschlammproduktion kann bei der Alkoholgärung zugegeben werden, auch wenn die Rohstoffe offenbar genügend Nährstoffe für das Hefewachstum enthalten.

Schematisch kann der Verfahrensablauf wie folgt beschrieben werden:

Zuckerhaltige und/oder stärkehaltige und/oder zellulosehaltige Rohstoffe werden zusammen mit behandelter Dünnschlempe unter eventuellem Zusatz von Wasser zur Maischeherstellung einem mechanischen, thermischen, enzymatischen Aufschluß unterworfen. Unter Zugabe von Gärorganismen, z.B. Hefe oder Bakterien, Harnstoff, sowie Einführen von Luft und/oder Sauerstoff und $CO_2$ wird die Gärung absatzweise oder kontinuierlich durchgeführt. Danach folgt die Abtrennung von Alkohol und Grobstoffen. Die dünne Schlempe wird einem Belebtschlammfermenter zugeführt, in dem kontinuierlich oder diskontinuierlich gearbeitet wird, und zwar unter Zugabe von Nährsalzen, Spurenelementen, Luft bzw. Sauerstoff, Säuren, Laugen oder Flüssiggülle. Die so behandelte Dünnschlempe wird wieder der Maischeherstellung zugeführt.

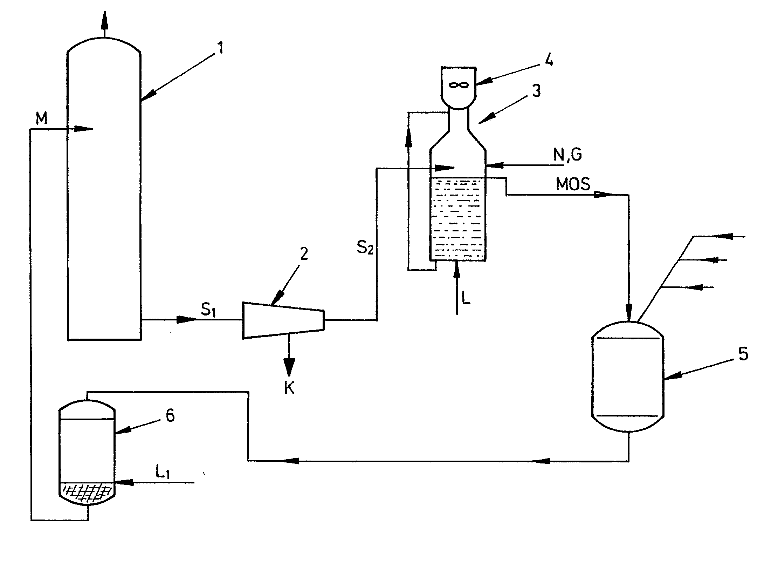

In der Zeichnung ist eine schematische Darstellung dieses Kreislaufes veranschaulicht.

Aus einer Destillierkolonne 1 wird durch eine Leitung Schlempe S1 in einen Dekanter 2 geleitet, dem die Grobstoffe als "Kuchen" K entnommen werden, während die Dünnschlempe S2 einem mit einem Kühler 4 versehenen Belebtschlamm- oder Bakterienfermenter 3 zugeführt wird, in den wahlweise Nährsalze, Spurenelemente, Säuren oder Laugen N, Luft L bzw. Sauerstoff, oder Flüssiggülle G eingeleitet werden. Die Mikroorganismen-Suspension MOS gelangt in eine Vorrichtung zur Maischeherstellung 5, die mit Enzymen, Wasser und Rohstoff für den Alkohol gespeist wird.

Das hier entstandene Produkt wird in einen belüfteten (Leitung L1) Alkohol-Fermenter 6 geleitet, in den auch Verzuckerungsenzyme dosiert werden können, aus dem dann Maische der Destillierkolonne 1 zugeführt wird.

Etwa 800 l Dünnschlempe pro Stunde wurden in einen Fermenter eingebracht, der 2,5 m Durchmesser und 5 m Höhe aufweist, und ständig mit 20 $m^3$ Mikroorganismenschlamm gefüllt war. Es wurde ständig mit etwa 150 $m^3$ Luft pro Stunde belüftet und der Inhalt wurde ständig durch einen über dem offenen Fermenter befindlichen Kühlturm auf 25 bis 40°C gekühlt. Harnstoff und Phosphate bzw. Ammoniumsulfat wurden in einer solchen Menge der Dünnschlempe im Fermenter zugeführt, daß stündlich 2 kg Stickstoff und 0,5 kg $P_2O_5$ für die Bakterien zur Verfügung standen.

Es empfiehlt sich jedoch einen Teil des Stickstoffes, beispielsweise 1 kg Harnstoff, pro Stunde nicht hier in den Fermenter, sondern in den Alkoholgärbottich zu geben.

Der Belebtschlamm-Fermenter enthält-vorzugsweise Belebtschlamm, der aus Anlagen für die Abwasserbehandlung von Kommunen oder Ernährungsindustrien bezogen werden kann.

Beim Fermentieren bzw. bei der Kühlung, verdunstete ein Teil des Wassers, so daß stündlich etwa 750 l Mikroorganismen-Suspension mit etwa 30 kg Trockenmassebzw. 12 kg Protein den Fermenter verließen, die ohne Zwischenbehandlung zum Einteigen des gemahlenen Rohstoffes, beispielsweise Mais, etwa 240 kg pro Stunde, benutzt wurden. Um wieder 1 $m^3$ Maischevolumen zu erreichen, mußten noch etwa 80 l Wasser oder Gülle ergänzt werden. Die Maische wurde wie üblich thermisch und enzymatisch behandelt, so daß süße Maische entstand, die in herkömmlicher Weise vergoren wurde.

Um mehr Alkohol und mehr Protein zu gewinnen, wurden in einen 24 $m^3$ fassenden Gärbottich stündlich etwa 5 $m^3$ Luft zur Aufrechterhaltung mikroaerober Verhältnisse eingeblasen. Beim Start einer neuen Gärung wurden etwa 500 l vergorene, aber ständig belüftete Maische im Bottich als Impfmaische belassen und stündlich wurden 1000 l süße Maische zugepumpt, bis der Bottich gefüllt war. Unter ständiger Weiterbelüftung war die Maische nach 2 Tagen vergoren. Die Wartezeit kann 1 bis 2 Tage dauern und richtet sich nach der Menge von zugesetzten Verzuckerungsenzymen.

Bei einer besonders vorteilhaften Ausgestaltung des erfindungsgemäßen Verfahrens werden die Verzuckerungsenzyme nicht vor der Gärung zugesetzt, sondern erst während dieser, und zwar in einer solchen Weise, daß ständig weniger als 2 g/l, vorzugsweise weniger als 0,2 g/l, Glucose bzw. Maltose oder Cellobiose feststellbar sind. Dazu muß erst 8 bis 12 Stunden nach dem Start einer neuen Gärung mit der Dosierung von Enzymen in den Gärbottich begonnenwerden.

Durch dieses Verfahren wird eine Mehrausbeute an Alkohol von etwa 5 bis 8 % gegenüber bekannter Betriebsweise und eine Mehrausbeute an Protein bis zu 12 kg/1000 Liter Maische je nach verwendetem Rohstoff erzielt werden. Es fällt keine Flüssigschlempe, sondern nur Kuchen an, wenn mit Getreide oder Trocken-Rohstoffen gearbeitet wird.

Anstelle der Zufuhr von Nährstoffen, Spurenelemente, Säuren oder Laugen können auch Flüssigabfälle aus der Tierhaltung, d.h. verflüssigter Kot oder Urin dem Belebtschlamm- oder Bakterienfermenter zugeführt werden, die insbesondere bei der Massentierhaltung anfallen und nicht sterilisiert werden müssen. Bei der Zugabe von Flüssiggülle ist die Menge der zugegebenen Flüssigkeit entsprechend zu beachten. Auf diese Weise kann die Flüssiggülle vorteilhaft verwertet und in wertvolle Futtermittel umgewandelt werden und dabei gleichzeitig das Problem der Beseitigung der insbesondere bei der Massentierhaltung in erheblichen Mengen anfallenden Flüssiggülle verringert werden.

Das Verfahren ist auch mit anderen alkoholbildenden Mikroorganismen als Hefe, beispielsweise dem Bakterium zymomonas mobilis durchführbar.

## Ansprüche

1. Verfahren zur Gewinnung von Alkohol und proteinangereicherter Schlempe aus zucker-, stärke- und/oder zellulosehaltigen Rohstoffen, bei dem die bei der Alkoholabtrennung anfallende und von Grobstoffen befreite dünne Schlempe biologisch mit Mikroorganismen behandelt wird, dabei in der Schlempe gelöste organische Stoffe abgebaut werden, die Mikroorganismen vermehrt werden und die so behandelte Schlempe bei der Herstellung der Maische für die Alkoholgärung benutzt wird,

**dadurch gekennzeichnet,**

daß die Mikroorganismen aus Belebtschlamm bestehen und daß die Schlempe nach der biologischen Behandlung einen biologischen Sauerstoffbedarf von weniger als 1000 Milligramm pro Liter aufweist.

2. Verfahren nach Anspruch 1, **dadurch gekennzeichnet,** daß zur Bildung einer Höchstmenge an Mikroorganismen, Nährsalze, Spurenelemente, Sauerstoff, Flüssiggülle oder andere nährstoffhaltige Tierabfälle dem Belebtschlamm zugesetzt werden.

3. Verfahren nach Anspruch 2, **dadurch gekennzeichnet**, daß ein Teil der für die Belebtschlammproduktion benötigten Nährstoffe, beispielsweise Harnstoff, bei der Alkoholgärung hinzugegeben wird.

4. Verfahren nach einem der vorangehenden Ansprüche, **dadurch gekennzeichnet**, daß der pH-Wert während der biologischen Behandlung auf 6 bis 8 eingestellt wird.

5. Verfahren nach einem der vorangehenden Ansprüche, **dadurch gekennzeichnet**, daß bei der Alkoholgärung ein Teil gärender Maische oder vergorener Maische als Impfmaische verwendet wird und bei Verwendung von stärke- oder zellulosehaltigen Rohstoffen unverzuckerte, an Verzuckerungsenzymen freie Maische zugegeben und Verzuckerungsenzyme dann in den Gärbottich dosiert werden, wenn in der gärenden Maische die Glucose und Maltosekonzentration bzw. Cellobiosekonzentration unter 5 Millimol pro Liter gesunken ist.

6. Verfahren nach Anspruch 5, **dadurch gekennzeichnet**, daß die Verzuckerungsenzyme zusammen mit restlichen Mengen an zulaufender Maische zugegeben werden.

7. Verfahren nach Anspruch 5 oder 6, **dadurch gekennzeichnet**, daß die Impfmaischmenge 1 bis 5 % der Endmenge beträgt und daß mit der Dosierung von Verzuckerungsenzymen 8 bis 12 Stunden nach dem Zusatz von unverzuckerter Maische begonnen wird.

8. Verfahren nach einem der vorangehenden Ansprüche, **dadurch gekennzeichnet**, daß bei konzentrierten Maischen ein mehrstufiges kontinuierliches Gärverfahren durchgeführt wird, bei dem der für die gewünschte Endkonzentration notwendige Zusatz von Wasser insgesamt zusammen mit einem Teil der konzentrierten Maische in einen ersten Gärbottich gegeben wird und daß die aus dieser ersten Stufe ablaufende gärende Maische in weiteren Stufen mit konzentrierter Maische versetzt und bei einer Glucose-, Maltose- oder Cellobiosekonzentration von weniger als 5 Millimol pro Liter vergoren wird.

9. Verfahren nach einem der vorangehenden Ansprüche, **dadurch gekennzeichnet**, daß die aus der Alkoholabtrennung in einer Destillierkolonne (1) kommende Rohschlempe (S₁) in einem Dekanter (K) von Feststoffen befreit wird, die Dünnschlempe (S₂) einem mit einem Kühler (4) versehenen Belebtschlamm- und Bakterienfermenter (3) zugeführt wird und unter Zusatz von Nährstoffen (N, G) mit Belebtschlamm aerob fermentiert wird und daß die derart behandelte Schlempe bei der Herstellung von frischer Maische mitverwendet wird.

10. Verfahren nach Anspruch 9, **dadurch gekennzeichnet**, daß die Maische in einen belüfteten Alkohol-Fermenter (6) geleitet wird, in den auch Verzuckerungsenzyme dosiert werden und aus dem Maische der Destillierkolonne (1) zugeführt wird.

**Claims**

1. Process for obtaining alcohol and protein-enriched spent wash from sugar-containing, starch-containing and/or cellulose-containing raw materials, in which the spent wash produced upon the separation of alcohol and which is freed of coarse materials is treated biologically with microorganisms, whereby organic materials dissolved in the spent wash are decomposed, the microorganisms are increased and the thus treated wash is used in the production of the mash for the fermentation of alcohol
characterised in that
the microorganisms consist of activated sludge and that the wash has a biological oxygen requirement of less than 1000 milligrammes per litre after the biological treatment.

2. Process according to Claim 1, characterised in that nutrient salts, trace elements, oxygen, liquid manure or other nutrient-containing animal waste is added to the activated sludge in order to form a maximum quantity of microorganisms.

3. Process according to Claim 2, characterised in that part of the nutrients required for the production of activated sludge, for instance urea, is added during the fermentation of alcohol.

4. Process according to one of the preceding Claims, characterised in that the pH value during the biological treatment is set to 6 to 8.

5. Process according to one of the preceding Claims, characterised in that during the fermentation of alcohol one part of fermenting mash or fermented mash is used as inoculation mash and when using starch-containing or cellulose-containing raw materials non-saccharified mash which is free of saccharification enzymes is added and saccharification enzymes are metered into the fermentation vat when the glucose and maltose concentration or cellobiose concentration in the fermenting mash has dropped to below 5 millimole per litre.

6. Process according to Claim 5, characterised in that the saccharification enzymes are added together with residual amounts of mash running in.

7. Process according to Claim 5 or 6, characterised in that the quantity of inoculation mash is 1 to 5% of the final quantity and that the metering of saccharification enzymes is begun 8 to 12 hours after the addition of non-saccharified mash.

8. Process according to one of the preceding Claims, characterised in that in concentrated mashes a multi-stage continuous fermentation process is carried out in which the total addition of water which is necessary for the desired final concentration is poured together with part of the concentrated mash into a first fermentation vat and that in additional stages concentrated mash is added to the fermenting mash emerging from this first stage and is fermented at a glucose, maltose or cellobiose concentration of less than 5 millimole per litre.

9. Process according to one of the preceding Claims, characterised in that the raw spent wash ($S_1$) coming from the alcohol separation in a distillation column (1) is freed of solids in a decanter (K), the spent wash ($S_2$) is fed to an activated sludge and bacteria fermenter (3) provided with a cooler (4) and is aerobically fermented with activated sludge with the addition of nutrients (N, G) and that the spent wash treated in this manner is also used in the production of fresh mash.

10. Process according to Claim 9, characterised in that the mash is passed into a ventilated alcohol fermenter (6) into which saccharification enzymes are also metered and from which mash is fed to the distillation column (1).

**Revendications**

1. Procédé de production d'alcool et de vinasse enrichie en protéine à partir de matières premières contenant du sucre, de l'amidon et/ou de la cellulose, dans lequel la vinasse claire formée au cours de la séparation d'alcool et exempte de substances grossières est biologiquement traitée par des micro-organismes, des substances organiques dissoutes dans la vinasse étant alors dégradées, les micro-organismes se multipliant et la vinasse ainsi traitée étant utilisée pour la préparation du moût destiné à la fermentation alcoolique, caractérisé en ce que les micro-organismes sont constitués d'une boue activée et en ce que la vinasse présente, après le traitement biologique, un besoin en oxygène biologique inférieur à 1.000 mg par litre.

2. Procédé suivant la revendication 1, caractérisé en ce que, pour la formation d'une quantité maximale de micro-organismes, on ajoute à la boue activée des sels nutritifs, des oligo-éléments, de l'oxygène, du purin liquide ou d'autres déchets animaux contenant des substances nutritives.

3. Procédé suivant la revendication 2, caractérisé en ce qu'une partie des substances nutritives nécessaires à la production de boue activée, par exemple de l'urée, est ajoutée au cours de la fermentation alcoolique.

4. Procédé suivant l'une des revendications précédentes, caractérise en ce que le pH est ajusté à une valeur de 6 à 8 pendant le traitement biologique.

5. Procédé suivant l'une des revendications précédentes, caractérisé en ce que, au cours de la fermentation alcoolique, une partie du moût fermentant cu du moût fermenté est utilisée comme moût d'inoculation et en ce que, lors de l'utilisation de matières premières contenant de l'amidon ou de la cellulose, on ajoute du moût non saccharifié, exempt d'enzymes de saccharification, et en ce que des enzymes de saccharification sont ensuite dosées dans la cuve de fermentation lorsque la concentration en glucose et en maltose ou respectivement la concentration en cellobiose dans le moût fermentant est descendue en dessous de 5 millimoles par litre.

6. Procédé suivant la revendication 5, caractérisé en ce que les enzymes de saccharification sont ajoutées conjointement à des quantités résiduaires de moût alimenté.

7. Procédé suivant l'une des revendications 5 et 6, caractérisé en ce que la quantité de moût d'inoculation est de 1 à 5 % de la quantité finale et en ce qu'on commence le dosage des enzymes de saccharification 8 à 12 heures après l'addition de moût non saccharifié.

8. Procédé suivant l'une des revendications précédentes, caractérise en ce que, dans le cas de moûts concentrés, on effectue un procédé de fermentation continu en plusieurs étapes dans lequel l'addition d'eau nécessaire à la concentration finale souhaitée est effectuée globalement dans une première cuve de fermentation conjointement à une partie du moût concentré et en ce que le moût fermentant sortant de cette première étape est additionné de moût concentré dans des étapes ultérieures et est fermenté à une concentration en glucose, maltose ou cellobiose inférieure à 5 millimoles par litre.

9. Procédé suivant l'une des revendications précédentes, caractérisé en ce que la vinasse brute (51) provenant de la séparation d'alcool dans une colonne de distillation est libérée, dans un dispositif de décantation (K), des substances solides, en ce que la vinasse claire ($S_2$) est amenée à un fermenteur à boue activée et à bactéries (3), pourvu d'un dispositif de refroidissement (4), et est fermentée en aérobiose avec la boue activée, avec addition de substances nutritives (N, G) et en ce que la vinasse ainsi traitée est utilisée conjointement pour la préparation de moût frais.

10. Procédé suivant la revendication 9, caractérisé en ce que le moût est amené dans un fermenteur à alcool (6) aéré dans lequel des enzymes de saccharification sont aussi dosées et à partir duquel du moût est amené à la colonne de distillation (1).